(19) Europäisches Patentamt
European Patent Office
Office européen des brevets

(11) **EP 1 961 365 A1**

(12) **EUROPEAN PATENT APPLICATION**
published in accordance with Art. 158(3) EPC

(43) Date of publication:
**27.08.2008 Bulletin 2008/35**

(51) Int Cl.:
*A61B 1/00* (2006.01)   *A61B 5/06* (2006.01)
*A61B 5/07* (2006.01)

(21) Application number: **06833904.3**

(22) Date of filing: **28.11.2006**

(86) International application number:
**PCT/JP2006/324134**

(87) International publication number:
**WO 2007/069483 (21.06.2007 Gazette 2007/25)**

(84) Designated Contracting States:
**DE FR GB**

(30) Priority: **16.12.2005 JP 2005362802**

(71) Applicant: **Olympus Corporation
Tokyo 151-0072 (JP)**

(72) Inventors:
• **SHIMIZU, Hatsuo
Tokyo 151-0072 (JP)**
• **NAKAMURA, Mikio
Tokyo 151-0072 (JP)**

(74) Representative: **Winter, Brandl, Fürniss, Hübner
Röss, Kaiser,
Polte Partnerschaft Patent- und
Rechtsanwaltskanzlei
Alois-Steinecker-Strasse 22
85354 Freising (DE)**

(54) **SYSTEM FOR DETECTING POSITION IN SUBJECT**

(57)    A medical capsule device (100) includes a first pad (109), an apparatus outside the body (200) includes a plurality of second pads (such as 201a), and at least one of the medical capsule device (100) and the apparatus outside the body (200) includes a modulating unit (106) which applies a voltage to the pad of one of the apparatuses, upon modulating a signal, and the other apparatus includes a demodulating unit (203) which demodulates the signal from an electric potential of the pad of the other apparatus, and moreover, includes a position-detection signal generating unit (111) for transmitting a signal for position detection, and a position calculating unit (204) which calculates a position of the medical capsule device (100), based on a magnitude of a signal strength of the signal for position detection which is demodulated from the electric potential at the plurality of second pads (such as 201a).

FIG. 1

**Description**

TECHNICAL FIELD

The present invention relates to an in-body position detecting system which transmits information of inside of a body to be examined, and position information of an apparatus in the body to be examined, between an apparatus which is disposed inside the body to be examined, and an apparatus which is disposed outside the body to be examined.

BACKGROUND ART

In recent years, in a field of examining and treating a body which is examined, particularly in-vivo examination and treatment, information related to a living body which is acquired inside the body, or near the body, is transmitted outside the body. Outside the body, it is possible to acquire in vivo information, particularly video information based on a signal received.

For example, as an apparatus introduced inside the body to be examined, it is possible to use a medical capsule device which includes an outer covering having a cylindrical shape with a base, and which can be introduced inside the body to be examined. A swallowable medical capsule device has an imaging function and a wireless communication function.

When the medical capsule device moves inside a body cavity, image data taken inside the body by the medical capsule device is transmitted one after another to an outside by wireless communication. The image data is stored in a memory which is provided outside. By taking along a receiving set which is equipped with the wireless communication function and a memory function, the body to be examined can move freely during a time after the medical capsule device is swallowed, till excreted. After the medical capsule device is excreted, a doctor or a nurse can diagnose upon displaying images of organs based on the image data which is stored in the memory.

Regarding such medical capsule device, for taking endoscope images of specific organ in the body to be examined for example, a medical capsule device which is provided with a function of performing position detection of the medical capsule device, inside the body to be examined, at a receiving set side has been proposed. As an example of a medical capsule device system equipped with this position detection function, a medical capsule device in which the wireless communication function integrated in the medical capsule endoscope is used, has been known. In other words, the receiving set which is disposed outside the body to be examined has a structure including a plurality of antenna elements. A wireless signal transmitted from the medical capsule device is received at the plurality of antenna elements. Further, it has a mechanism of detecting a position of the medical capsule device in the body to be examined, based on a difference in receiving signal strength at each antenna element (refer to Japanese Patent Application Laid-open Publication No. 2003-19111 for example).

Furthermore, a structure which searches a position of a signal source inside the body has also been proposed (refer to Japanese Patent Application Laid-open Publication No. 2005-192631 for example). In the Japanese Patent Application Laid-open Publication No. 2005-192631, an apparatus introduced inside the body to be examined includes a magnetic field generating unit. Moreover, a constant magnetic field is output to an outside of the body to be examined. The magnetic field generating unit derives a position of the apparatus introduced inside the body to be examined, based on strength of the constant magnetic field output by the magnetic field generating unit, from which a noise magnetic field component is eliminated.

In a structure such as the one disclosed in Japanese Patent Application Laid-open Publication No. 2003-19111, by an antenna element disposed outside the body, a receiving signal strength distribution of a signal received from a signal source in the body is calculated. In this structure, a dead zone is developed according to a direction of an electric field. Therefore, it is not possible to detect a position of a signal source inside the body accurately.

Moreover, at the time of communicating with the outside of the body by electric wave, the following problems (1), (2), and (3) occur. Therefore, there is a substantial strain on a patient.

(1) A frequency which can be used is restricted due to regulations. Therefore, it is difficult to select a frequency appropriate for communication between the inside and outside of the body.

(2) It is necessary to install an antenna inside and outside the body for transceiving (transmitting and receiving). Here, the electric wave is affected such as being attenuated in the body. Therefore, the antennas to be installed outside the body have to be large size and in multiple number. As a result of this, there is a substantial strain on the patient.

(3) As it has been mentioned above, the electric waves are affected such as being attenuated in the body. Therefore, a high electric-wave output is necessary. Consequently, it is difficult to reduce a size of an in vivo and an in vitro apparatus. As a result of this, there is a substantial strain on the patient.

Moreover, in Japanese Patent Application Laid-open Publication No. 2005-192631, the position of the apparatus introduced inside the body to be examined is calculated from strength of a magnetic field. In this structure, it is necessary to provide the magnetic field generating unit which is included in the apparatus introduced inside the body to be examined, and a unit for detecting the magnetic field. Therefore, the structure becomes complicated, and there is an increase in a size of the apparatus.

The present invention is made in view of the abovementioned issues, and an object of the present invention is to provide an in-body position detecting system which is capable of detecting accurately and without a dead zone due to a direction of the electric field, a position without a need of disposing an antenna, a magnet, and a magnetic field detecting sensor in an apparatus outside the body to be examined, and which reduces the strain on the patient as a size of the apparatus inside the body to be examined can be made smaller.

DISCLOSURE OF THE INVENTION

For solving the issues mentioned above, and achieving the object, according to the present invention, it is possible to provide an in-body position detecting system, including
an apparatus introduced inside the body to be examined which is introduced inside the body, and
an apparatus outside the body which is disposed outside the body to be examined, and performs a communication with the apparatus introduced inside the body to be examined.

The apparatus introduced inside the body to be examined includes at least a first pad, and the apparatus outside the body includes a plurality of second pads.

For performing transceiving of a signal between the first pad and the second pad, at least one of the apparatus introduced inside the body to be examined and the apparatus outside the body includes a modulating unit which applies a voltage to the pad of one of the apparatuses upon modulating the signal, and the other apparatus includes a demodulating unit which demodulates the signal based on a change in an electric potential of the pad of the other apparatus.

The in-body position detecting system further includes
a signal generating unit which transmits a signal for position detection, and
a position calculating unit which calculates a position of the apparatus introduced inside the body to be examined, based on a magnitude of signal strength of the signal for position detection which is demodulated based on the change in the electric potential at the plurality of second pads.

Moreover, according to another aspect of the present, it is possible to provide an in-body position detecting system, including
an apparatus introduced inside the body to be examined which is introduced inside the body to be examined, and
an apparatus outside the body which is disposed outside the body, and performs a communication with the apparatus introduced inside the body to be examined.

The apparatus introduced inside the body to be examined includes at least a first pad, and
the apparatus outside the body includes a plurality of second pads.

For performing transceiving of a signal between the first pad and the second pad, at least one of the apparatus introduced inside the body to be examined and the apparatus outside the body includes a modulating unit which applies a voltage to the pad of one of the apparatuses upon modulating the signal, and the other apparatus includes a demodulating unit which demodulates the signal based on a change in an electric potential of the pad of the other apparatus.

The in-body position detecting system further includes
a signal generating unit which transmits a signal for position detection, and
a position calculating unit which calculates a position of the apparatus introduced inside the body to be examined, based on a magnitude of signal strength of the signal for position detection which is demodulated based on the change in the electric potential at the plurality of second pads.

At least one of the second pads included in the apparatus outside the body is a pad which becomes a reference for position detection, and
the pad which becomes the reference has a position adjusting section for adjusting a relative position with respect to a predetermined position of the body to be examined.

Moreover, according a preferable aspect of the present invention, it is desirable that the signal generating unit transmits the signal for position detection upon synchronizing with in vivo information signal.

Furthermore, according to another preferable aspect of the present invention, it is desirable that the signal generating unit, when not transmitting the in vivo information signal, transmits the signal for position detection.

According to still another preferable aspect of the present invention, it is desirable that the in vivo information signal is a video signal, and the signal generating unit transmits the signal for detection in an interval of a blank signal for a vertical synchronization of the video signal.

Moreover, according to still another preferable aspect of the present invention, it is desirable that the signal generating unit transmits the signal for position detection upon multiplexing with the in vivo information signal.

Furthermore, according to still another preferable aspect of the present invention, it is desirable that the apparatus introduced inside the body to be examined includes an imaging section which takes images of a portion to be examined of the body to be examined, and outputs at least a video signal, and the apparatus outside the body demodulates the video signal, and the signal for position detection is superimposed on the video signal.

According to still another preferable aspect of the present invention, it is desirable that the signal for position detection is generated by using a clock signal for control of at least one of the apparatus introduced inside the body to be examined and the apparatus outside the body.

Moreover, according to still another preferable aspect of the present invention, it is desirable that at least one of the second pads included in the apparatus outside the body is a pad which becomes a reference for the position detection.

BRIEF DESCRIPTION OF THE DRAWINGS

Fig. 1 is a diagram showing an overall structure of an in-body position detecting system according to a first embodiment of the present invention;
Fig. 2 is a diagram showing an external structure of a medical capsule device in the first embodiment;
Fig. 3 is a diagram showing functional blocks of the medical capsule device of the first embodiment;
Fig. 4 is a diagram showing functional blocks of an apparatus outside the body of the first embodiment;
Fig. 5 is a diagram showing a cross-sectional structure of a pad of the apparatus outside the body of the first embodiment;
Fig. 6 is a diagram showing a positional relationship of the medical capsule device and the pad;
Figs. 7A, 7B, and 7C are flowcharts showing a flow of a signal in the first embodiment;
Figs. 8A, 8B, and 8C are flowcharts showing a flow of position detection in the first embodiment;
Figs. 9A, 9B, and 9C are flowcharts showing a flow of posture detection in the first embodiment;
Fig. 10 is a diagram showing an external structure of another medical capsule device in the first embodiment;
Fig. 11 is a diagram showing functional blocks of a medical capsule device of a second embodiment;
Figs. 12A and 12B are diagrams showing a body, and a pad which becomes a reference for the in-body position detecting system according to a third embodiment of the present invention;
Fig. 13 is a diagram showing a schematic structure of a pad which becomes a reference, of the third embodiment; and
Fig. 14 is a diagram showing a cross-sectional structure of the pad which becomes a reference, of the third embodiment.

BEST MODE FOR CARRYING OUT THE INVENTION

Embodiments of an in-body position detecting system according to the present invention will be described below in detail by referring to diagrams. However, the present invention is not restricted to these embodiments.

(First embodiment)

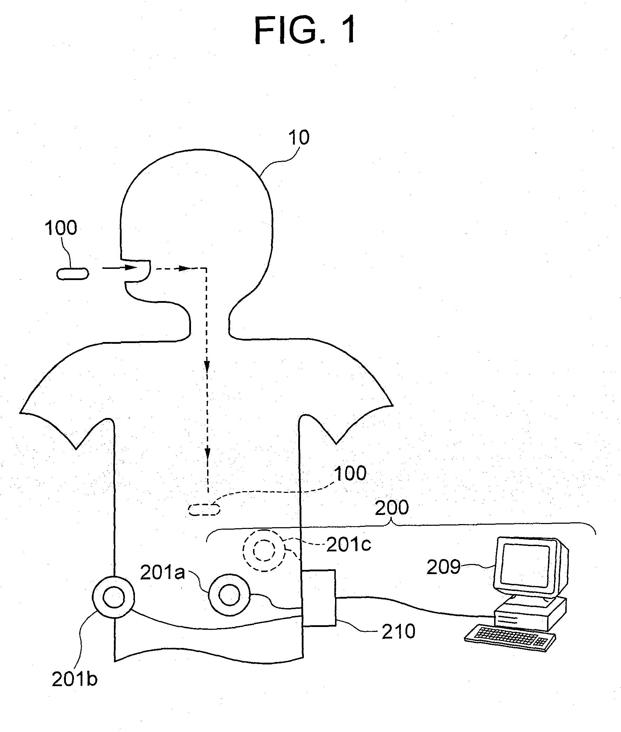

Fig. 1 is a diagram showing a schematic structure of an in-body position detecting system. A living body 10 as a body to be examined and a case of acquiring in vivo information of a patient is shown. A medical capsule device 100 has a function of moving with a peristaltic motion inside an organ such as a stomach and a small intestine and take images one after another, during an observation time from being swallowed for observation (examination) from a mouth by a patient till discharged out naturally from the body.

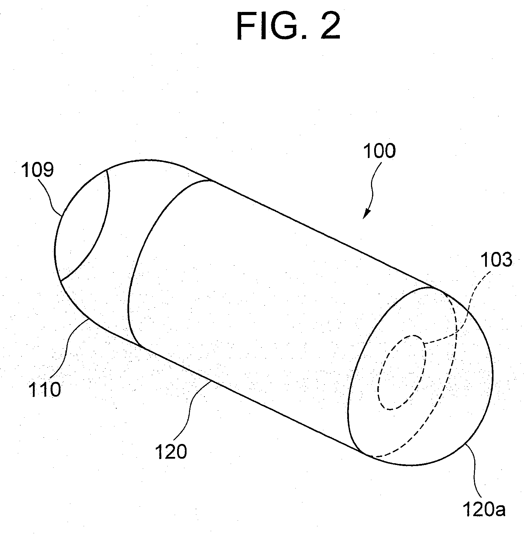

Fig. 2 shows a schematic external structure of the medical capsule device 100. The medical capsule device 100 corresponds to the apparatus introduced inside the body to be examined. Moreover, the medical capsule device 100 includes an outer covering 120 having a cylindrical shape with a base, and which can be introduced in the body 10. Furthermore, a first pad 109 which will be described later is formed on a surface of the medical capsule device 100. A CCD (charge coupled device) 103 is formed on a side opposite to a side on which the first pad 109 is formed.

Image data which is taken in the body by the medical capsule device 100 during observation by the movement in the organ is transmitted one after another to an apparatus outside the body 200 which is in vitro, by a communication procedure which will be described later. The medical capsule device 100 and the apparatus outside the body 200 form the in-body position detecting system. First of all, a structure of the medical capsule device 100 will be described, and then a structure of the apparatus outside the body 200 will be described.

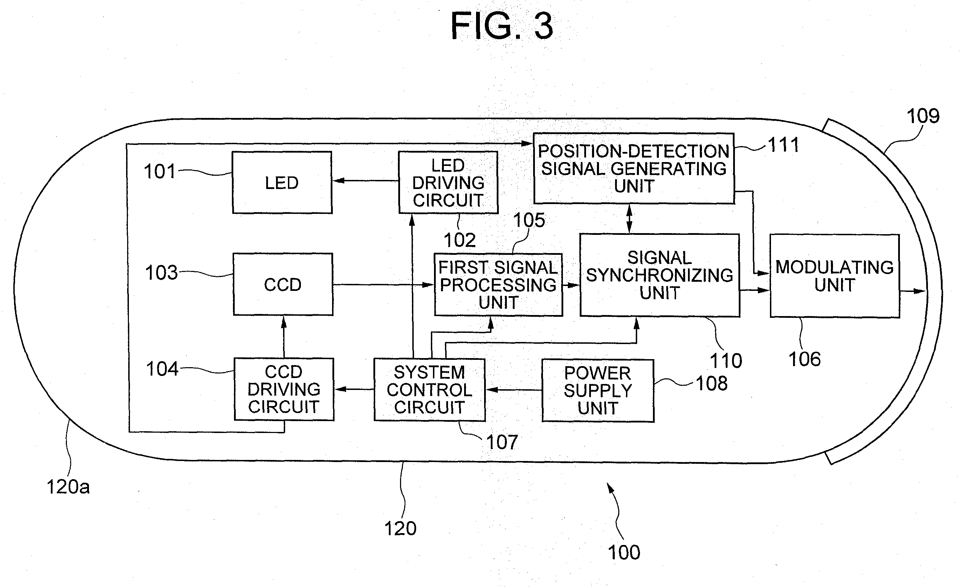

Fig. 3 shows functional blocks of the medical capsule device 100. The medical capsule device 100 includes an LED (light emitting diode) 101 for illuminating an imaging area at the time of imaging inside the living body 10, an LED driving circuit 102 which controls a driving of the LED 101, and a CCD 103 which takes images of the area illuminated in the body by the LED 101. Moreover, the medical capsule device 100 includes a CCD driving circuit 104 which controls a driving of the CCD 103, a first signal processing unit 105 which processes image data (video signal) taken by the CCD 103, a position-detection signal generating unit 111 which generates a signal for position detection for showing a position of the medical capsule device 100, a signal synchronizing unit 110 for synchronizing an in vivo information signal and a signal for position detection, a modulating unit 106 which modulates the in vivo information signal and the signal for position detection, a first pad 109 to which a modulated voltage from the modulating unit 106 is applied, and a system control circuit 107 which controls operations of the LED driving circuit 102, the CCD driving circuit 104, the first signal processing unit 105, the position-detection signal generating unit 111, the signal synchronizing unit 110, and the modulating unit 106. Moreover, a power supply unit 108 supplies an electric power to each unit and circuit etc. in the medical capsule device 100.

The CCD 103 acquires in vivo information such as image information inside the living body 10. The CCD 103 corresponds to the imaging section, and has a function as an in vivo information sensor. Apart from the CCD 13, CMOS (complementary metal oxide semiconductor) can be used as the imaging section. At least a part of a window 120a on the outer covering of the medical capsule device 100 is formed of a material such as a transparent material. The CCD 103 takes images of the living body 10 through the window 120a.

The CCD 103 is connected to the CCD driving circuit 104. The CCD driving circuit 104 outputs to the CCD 103, an actuating signal and a clock signal for acquiring the in vivo information. The CCD 103 is connected to the first signal processing unit 105. The first signal processing unit 105 has a function as an in vivo information processing unit. The first signal processing unit 105 includes circuits such as a data compression circuit and an image converting circuit for output from the CCD 103. Moreover, the first signal processing unit 105 generates and outputs an in vivo information signal from an output signal of the CCD 103.

The CCD driving circuit 104 and the first signal processing unit 105 are connected to the signal synchronizing unit 110 via the system control circuit 107. Moreover, the position-detection signal generating unit 111 generates the signal for position detection for detecting the position of the medical capsule device 100, based on the clock signal generated by the CCD driving circuit 104. Here, the clock signal may be used as it is or the clock signal may be subjected to frequency division. The signal for position detection may be generated based on a clock signal for control of the apparatus outside the body 200 and the medical capsule device 100.

The position-detection signal generating unit 111 is connected to the signal synchronizing unit 110. The signal synchronizing unit 110 outputs the signal for position detection to the modulating unit 106 in synchronism with the in vivo information signal.

Moreover, the in vivo information signal, for example when a frame rate of the video signal is slow, may transmit the position detection signal when the in vivo information signal is not transmitted. For example, when the in vivo information signal is a video signal, there exists a blank interval (H blank and V blank) for signal synchronization in a horizontal direction and a vertical direction. Moreover, during the blank interval, the signal for position detection may be transmitted in the interval of a blank signal for the vertical synchronization.

The modulating unit 106 modulates the in vivo information signal and the signal for position detection, and applies to the first pad 109.

The first pad 109 is formed of a material such as copper (Cu) and nickel (Ni), which does not include any substance harmful to the living body. In general, the first pad 109 is formed of a material such as platinum (Pt) and gold (Au).

The first pad 109 is formed on an outer surface of the medical capsule device 100. An inside of the medical capsule device 100 is a sealed structure. The first pad 109 is connected to the modulating unit 106 while maintaining the sealed state of the medical capsule device 100. The first pad 109 and the modulating unit 106 are formed by sealing a through hole by filling a material such as a resin and a metal, upon being connected by passing through the through hole (not shown in the diagram) of the medical capsule device 100. Next the apparatus outside the body 200 will be described.

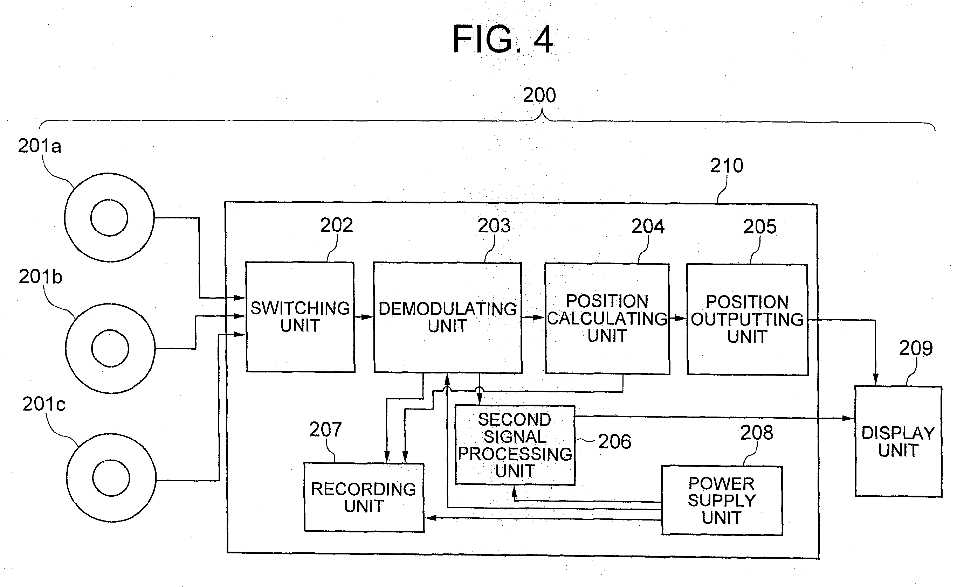

Fig. 4 shows functional blocks of the apparatus outside the body 200. Second pads 201a, 201b, and 201c are installed on a surface of the living body 10. Moreover, the second pads 201a, 201b, and 201c are connected to a switching unit 202 in a portable unit 210. The portable unit 210, for example, is mounted near a waist belt of the living body 10.

The portable unit 210 includes the switching unit 202, a demodulating unit 203, a position calculating unit 204, a position outputting unit 205, a second signal processing unit 206, a recording unit 207, and a power supply unit 208.

By applying to the first pad 105, a voltage in which the in vivo information signal and the signal for position detection are modulated, there occurs to be a change in an electric potential on a surface of the second pads 201a, 201b, and 201c. The demodulating unit 203 demodulates the in vivo information signal and the signal for position detection, based on the change in the electric potential of the surface of the second pads 201a, 201b, and 201c.

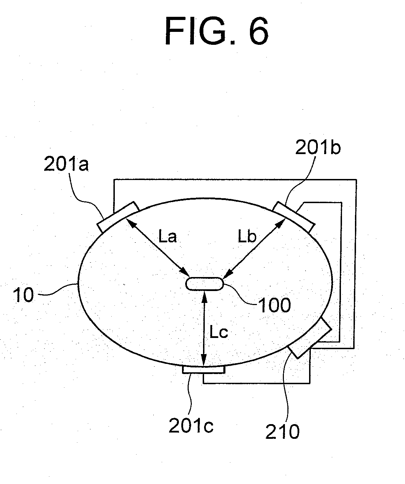

The apparatus outside the body 200 includes a plurality, such as three second pads 201a, 201b, and 201c. Fig. 6 shows a cross section of the living body 10. The second pads 201a, 201b, and 201c are attached to the surface of the living body 10, at positions at substantially equal intervals.

The description will be continued coming back to Fig. 4. The plurality of second pads 201a, 201b, and 201c are connected to the switching unit 202. The switching unit 202 selects one of the change in the electric potential of the second pads 201a, 201b, and 201c, and outputs to the demodulating unit 203.

The demodulating unit 203, based on the change in the electric potential of the second pads 201a, 201b, and 201c, outputs the in vivo information signal to the second signal processing unit 206. The second signal processing unit 206 is a circuit such as a decompression circuit for compressed data, and correction / enhancing circuit of the image information. The second signal processing unit 206 performs a signal processing for acquiring the required in vivo information, based on the in vivo information signal which is demodulated by the demodulating unit 203.

Moreover, the second signal processing circuit 206 is connected to a display unit 209. The display unit 209 is a monitor such as a liquid crystal display. The display unit 209 displays the in vivo information which is processed in the second signal processing unit 206. In Fig. 1, the display unit 209 is not provided on the portable unit 210, but is provided else where. However, without restricting to this, the structure may be such that the display unit 209 is provided on the portable unit 210.

The recording unit 207 is connected to the demodulating unit 203 or to the second signal processing unit 206. The recording unit 207 includes a memory such as a semiconductor memory. The recording unit 207 records and stores three-dimensional position information which is calculated by the position calculating unit 204 based on a procedure which will be described later, and the in vivo information which is processed in the second signal processing unit 206.

Moreover, the power supply unit 208 supplies the electric power to the demodulating unit 203, the position calculating unit 204, the position outputting unit 205, the second signal processing unit 206, and the recording unit 207.

Furthermore, the second pads 201a, 201b, and 201c are formed of a material such as copper (Cu) and nickel (Ni), which does not include any substance harmful to the living body. In general, the second pads 201a, 201b, and 201c are formed of a material such as platinum (Pt) and gold (Au).

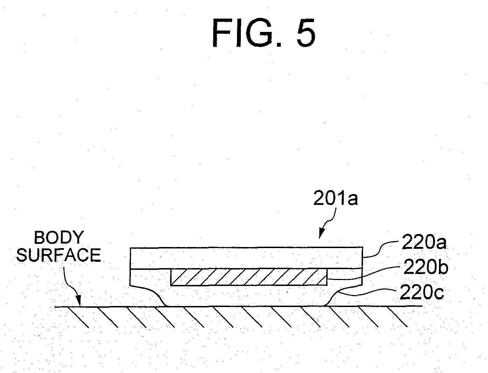

Fig. 5 shows a cross-sectional structure of the second pad 201a. The other pads 201b and 201c have structure similar to the second pad 201a. Since the second pad 201a makes a close contact with the body surface, the second pad 201a has a structure in which a thin film 220b made of a material such as platinum (Pt) and gold (Au) is sandwiched by a substrate 220a such as a resin film and a ribbon. Furthermore, a portion which makes a contact with the living body surface is formed of an insulating thin-film 220c made of a material such as a silicon resin. It is desirable that a thickness of the insulating thin-film 220c which makes a contact with the living body is not more than 1 mm so that the electric potential of the surface of the living body can be detected at the second signal processing unit 206. Moreover, a gel or oil may be applied between the surface of the living body 10 and the second pad 201a. Accordingly, a degree of an adhesion between the second pad 201a and the living body surface can be improved.

Thus, in this embodiment, since the information communication which is independent of an electric current is performed, the second pads 201a, 201b, and 201c can be let to have an insulating structure. Therefore, a safety of the living body 10 can be improved.

Next, the position detection of the medical capsule device 100 will be described. The demodulating unit 203 outputs to the position calculating unit 204 the signal for position detection which is demodulated, from each of the plurality of second pads 201a, 201b, and 201c. When the demodulating unit 203 exclusively for each of the second pads 201a, 201b, and 201c is connected to the second pads 201a, 201b, and 201c respectively, the switching unit 202 may not be provided.

The position calculating unit 204 compares the signal strength of the signal for position detection which is modulated from the three second pads 201a, 201b, and 201c respectively. Accordingly, the position calculating unit 204 calculates a three-dimensional position of the medical capsule device 100 in the living body 10. Details of a procedure for position calculation will be described later.

When the signal strength of all the signals for position detection which are modulated, from the second pads 201a, 201b, and 201c respectively is same, the medical capsule device 100 exists at a position which is equidistant from these three second pads 201a, 201b, and 201c. Whereas, when the signal strength of the signals for position detected which are modulated, from the second pads 201a, 201b, and 201c is different, the position calculating unit 204 calculates such that a ratio of the signal strength of the signal for position detection is replaced with a ratio of the distance between the plurality of second pads 201a, 201b, and 201c, and the medical capsule device 100. Accordingly, it is possible to calculate three-dimensionally the position of the medical capsule device 100. Moreover, the position calculating unit 204 outputs to the position outputting unit 205, the position information of the medical capsule device 100 in the living body 10.

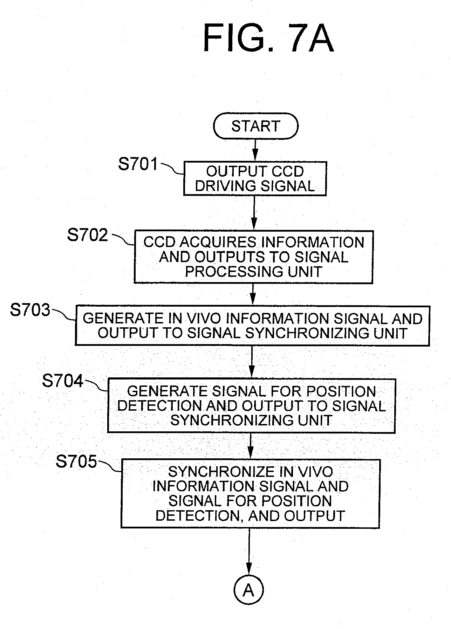

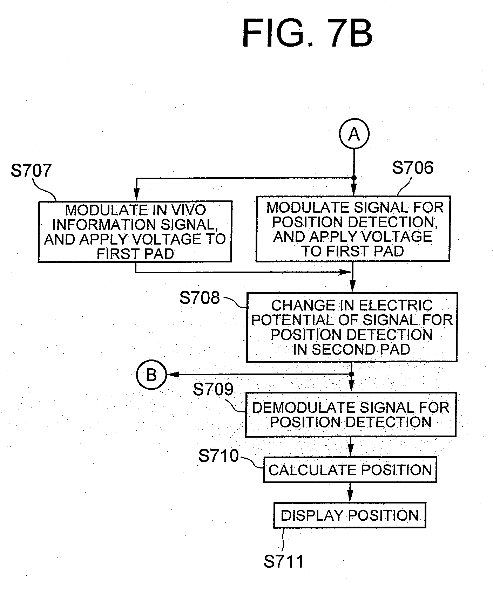

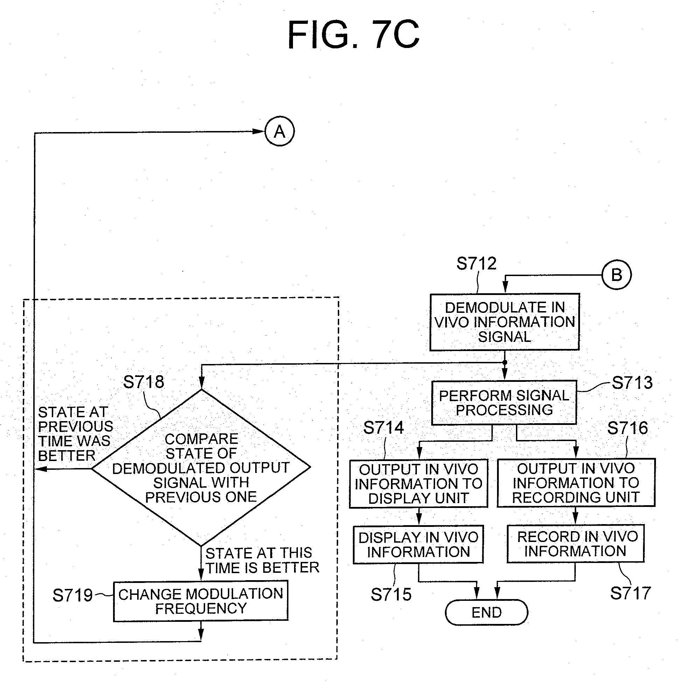

Figs. 7A, 7B, and 7C are flowcharts showing a procedure of communicating the in vivo information signal and the signal for position detection. At step S701, the CCD driving circuit 104 outputs a driving signal to the CCD 103. At step S702, the CCD 103 acquires the in vivo information (imaging). Next, the CCD 103 outputs the in vivo information acquired, to the first signal processing unit 105.

At step S703, the first signal processing unit 105 generates the in vivo information signal from an output signal of the CCD 103. Further, the first signal processing unit 105 outputs the in vivo information signal generated, to the signal synchronizing unit 110.

At step S704, the position-detection signal generating unit 111 generates the signal for position detection for detecting the position of the medical capsule device 100, based on a clock signal which is generated by the CCD driving circuit 104, and outputs to the signal synchronizing unit 110.

At step S705, the signal synchronizing unit 110 synchronizes the in vivo information signal and the signal for position detection, and outputs to the modulating unit 106.

At step S706, the modulating unit 106 modulates the signal for position detection. Further, the modulating unit 106 applies to the first pad 109, a voltage in accordance with the modulated output signal.

At step S707, the modulating unit 106 modulates the in vivo information signal. The modulating unit 106 applies to the first pad, a voltage in accordance with the modulated in vivo information signal.

According to the voltage applied to the first pad 109, which has modulated the in vivo information signal and the position detection signal, an electric potential of the surface of the second pads 201a, 201b, and 201c changes.

At step S708, the electric potential of the surface of the second pads 201a, 201b, and 201c changes. Further, the switching unit 202 switches upon selecting, any one of the second pads 201a, 201b, and 201c.

At step S709, the demodulating unit 203 demodulates the signal for position detection, based on the change in the potential of the surface of the second pads 201a, 201b, and 201c. At step S710, the position calculating unit 404, calculates the three-dimensional position of the medical capsule device 100, by a procedure which will be described later. At step S711, the position outputting unit 205 displays the position information, which is calculated.

Moreover, at step S712, the demodulating unit 203 demodulates the in vivo information signal, based on the change in the electric potential of the surface of the second pads 201a, 201b, and 201c. Further, the demodulating unit 203 outputs the output signal which is demodulated, to the second signal processing unit 206.

At step S713, the second signal processing unit 203 performs a signal processing for acquiring the necessary in vivo information from the in vivo information signal.

At step S714, the second signal processing unit 206 outputs to the display unit 209 the in vivo information which is acquired by the signal processing. At step S715, the display unit 209 displays the in vivo information.

Moreover, at step S716, the second signal processing unit 206 outputs to the recording unit 207, the in vivo information which is acquired by the signal processing. At step S717, the recording unit 207 records and stores the in vivo information. Furthermore, the recording unit 207 may be structured to be capable of recording and storing the signal for the position detection.

Next, an optimization of a modulation frequency will be described below. Based on a state (S/N ratio) of the output signal from the second signal processing unit, which is demodulated by the demodulating unit 203, it is possible to determine a modulation frequency when the modulating unit has applied to the first pad 109, the voltage upon modulating the output signal of the first signal processing unit 105.

For example, with an initial modulation frequency as a reference, the modulation frequency is changed to a lower side and a higher side of the initial modulation frequency, by the modulating unit 106. The initial modulation frequency is found by experiments etc., and means a frequency at which the state of the output signal of the second signal processing unit 206 is favorable in general.

Further, the state of the output signal of the second signal processing unit 206 which is demodulated by the demodulating unit 203, such as a frequency at which the S/N ratio etc. is improved, is determined as an optimum frequency.

Moreover, regarding the change of the modulation frequency, the frequency to be changed may be determined randomly, or the modulation frequency may be adjusted promptly to be the optimum modulation frequency by also using a so-called mountain climbing method (method of steepest descent). Apart from this, the frequency to be changed can be determined by using any algorithm.

Such a procedure for determining the optimum frequency will be described by referring to the flowchart in Figs. 7A, 7B, and 7C. At step S718, the state (S/N ratio) of the output signal of the second signal processing unit 206 which is demodulated by the demodulating unit 203 is compared with a previous state. When the present state is better than the previous state, at step S719, the modulation frequency is changed to the frequency at present. Next, the process returns to step S706. When the previous state is better than the present state, process returns to step S706. Thus, in Fig. 7C, procedure of a portion enclosed by dotted lines corresponds to an optimization procedure of the modulating frequency.

According to this, it is possible to reduce an effect of an individual variation of the living body 10, and a difference in a state of the living body according to that day and time, and to realize even more favorable communication between the medical capsule device 100 and the apparatus outside the body 200.

(Detailed procedure for position calculation)

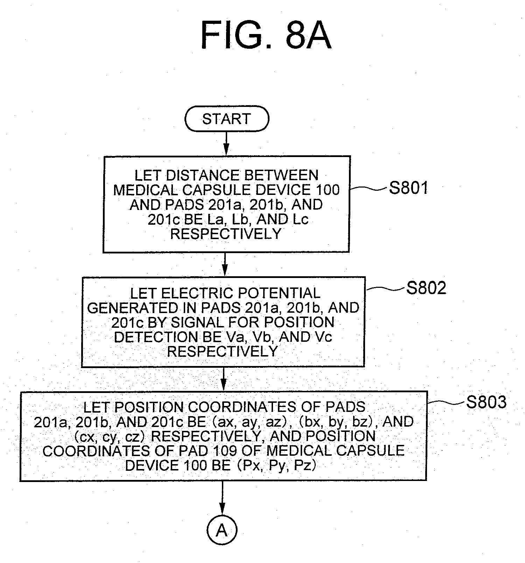

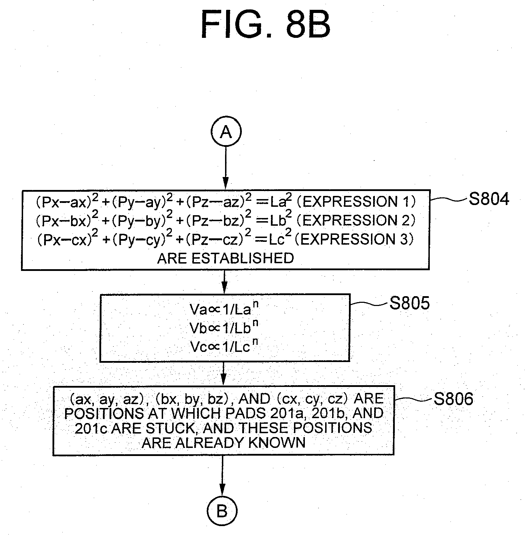

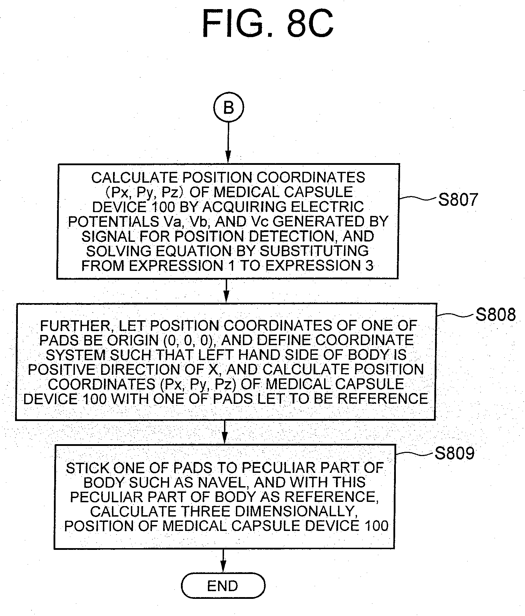

Next, details of the position detection of the medical capsule device 100 will be described below. Figs. 8A, 8B, and 8C are flowcharts showing a detail procedure of position detection. At step S801, the distance between the medical

7

capsule device 100 and each of the three second pads 201a, 201b, and 201c is let to be La, Lb, and Lc respectively (refer to Fig. 6).

At step S802, an electric potential generated in each of the three second pads 201a, 201b, and 201c by the signal for position detection is let to be Va, Vb, and Vc respectively. At step S803, position coordinates of the second pad 201a are let to be (ax, ay, az), position coordinates of the second pad 201b are let to be (bx, by, bz), and position coordinates of the second pad 201c are let to be (cx, cy, cx). Moreover, position coordinates of the first pad 109 are let to be (Px, Py, Pz).

At step S804, the following expressions (1), (2), and (3) are established.

$$(Px - ax)^2 + (Py - ay)^2 + (Pz - az)^2 = La^2 \quad ...(1)$$

$$(Px - bx)^2 + (Py - by)^2 + (Pz - bz)^2 = Lb^2 \quad ...(2)$$

$$(Px - cx)^2 + (Py - cy)^2 + (Pz - cz)^2 = Lc^2 \quad ...(3)$$

At step S805,

$$Va \propto 1/La^n$$

$$Vb \propto 1/Lb^n$$

$$Vc \propto 1/Lc^n$$

are established.

At step S806, the position coordinates (ax, ay, az), (bx, by, bz), and (cx, cy, cz) are positions at which the second pads 201a, 201b, and 201c are attached respectively, and are already known.

At step S807, the electric potentials Va, Vb, and Vc generated by the signal for position detection, are acquired. Further, by substituting these electric potentials in expressions (1), (2), and (3), the equations are solved. Accordingly, it is possible to calculate the position coordinates (Px, Py, Pz) of the medical capsule device 100.

At step S808, further, position coordinates of any of the three second pads 201a, 201b, and 201c are let to be an origin (0, 0, 0). Moreover, for example, a coordinate system in which a left hand side of the living body 10 is let to be a positive direction is defined. Accordingly, it is possible to calculate the position coordinates (Px, Py, Pz) of the medical capsule device 100 for which any one of the second pads is let to be a reference.

At step S809, any one of the second pads 201a, 201b, and 201c is attached to a peculiar part of a living body such as a navel of the living body 10. Accordingly, it is possible to calculate the three-dimensional position of the medical capsule device 100 letting the peculiar portion of the living body to be the reference.

(Posture detection)

According to the procedure mentioned above, it is possible to calculate the three-dimensional position coordinates in the living body 10 of the medical capsule device 100. Moreover, even more preferably, it is desirable to be able to detect a posture of the medical capsule device 100. Accordingly, it is possible to identify more accurately a part inside the living body 10 which is being observed by the CCD 103.

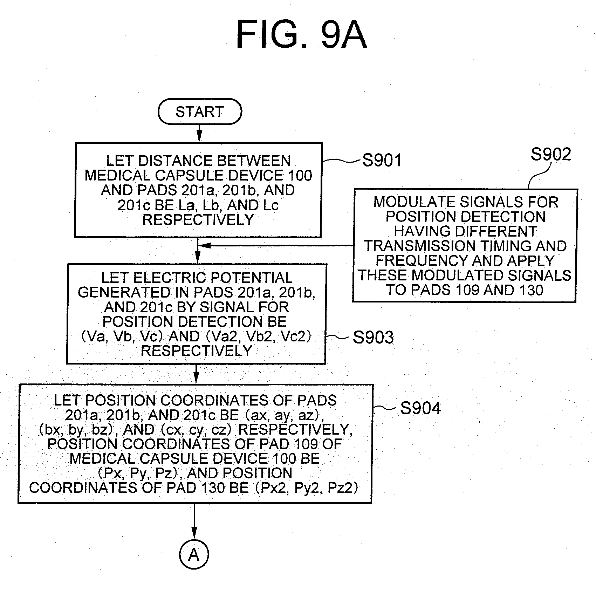

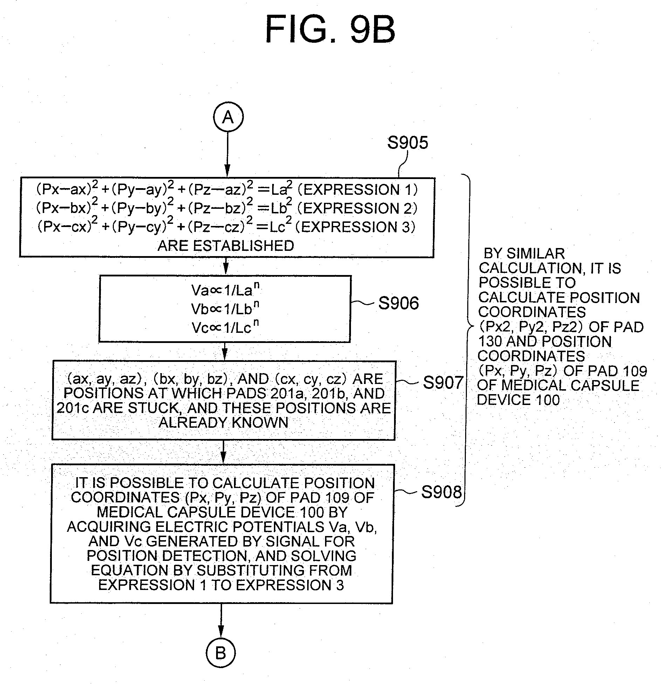

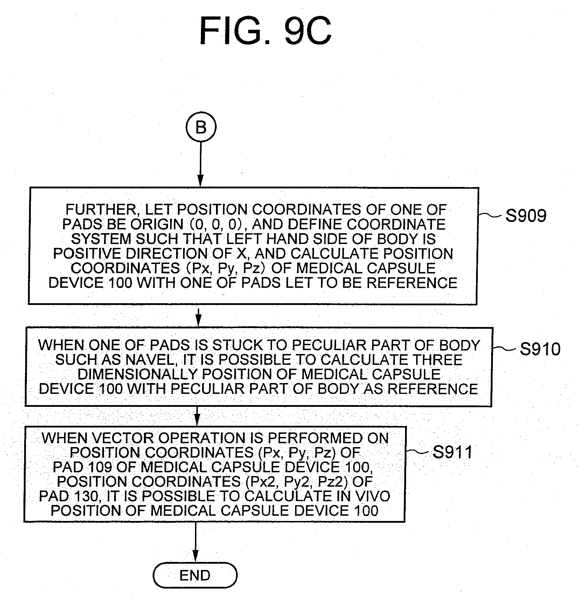

Figs. 9A, 9B, and 9C are flowcharts of a procedure of posture detection. At step S901, the distance between the medical capsule device 100 and each of the three second pads 201a, 201b, and 201c is let to be La, Lb, and Lc respectively (refer to Fig. 6).

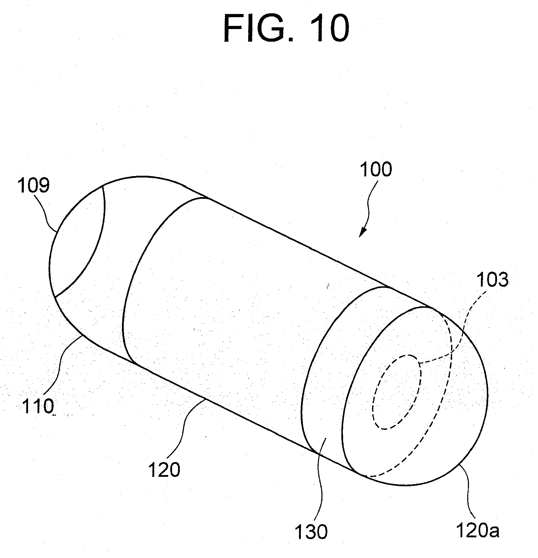

Moreover, at the time of performing the posture detection, as shown in Fig. 10, the medical capsule device 100 includes a pad 130 in addition to the pad 109. A structure and a function of the pad 130 are same as of the pad 109.

The description will be continued upon coming back to Fig. 9A. At step S902, a signal for position detection having different transmission timing and frequency is modulated and applied to each of the pad 109 and pad 130.

At step S903, the electric potential generated in each of the three second pads 201a, 201b, and 201c due to the signal for position detection from the pad 109 is let to be Va, Vb, and Vc respectively. Moreover, the electric potential generated in each of the three pads 201a, 201b, and 201c due to the signal for position detection from the pad is let to be Va2, Vb2, and Vc2 respectively.

At step S904, the position coordinates of the second pad 201a are let to be (ax, ay, az), the position coordinates of the second pad 201b are let to be (bx, by, bz), and the position coordinates of the second pad 201c are let to be (cx, cy, cz). Moreover, the position coordinates of the pad 109 are let to be (Px, Py, Pz), and position coordinates of the pad 130 are let to be (Px2, Py2, Pz2).

Procedure from step S905 to step S910 is similar to the procedure from step S804 to step S809 in Figs. 8B and 8C. Therefore, the description to be repeated will be omitted.

By a procedure similar to the position detection, the position (Px, Py, Pz) of the pad 109 of the medical capsule device 100 is calculated. Next, by a procedure similar to the position detection, the position (Px2, Py2, Pz2) of the pad 130 of the medical capsule device 100 is calculated.

At step S911, the position (Px, Py, Pz) of the pad 109 and the position (Px2, Py2, Pz2) of the pad 130 are subjected to a vector operation. Accordingly, it is possible to detect the posture of the medical capsule device 100.

By detecting the posture of the medical capsule device 100, it is possible to specify a position of a diseased part more accurately.

As it has been described above, according to this embodiment, the medical capsule device 100 and the apparatus outside the body 200 can communicate the in vivo information and the position information to the outside of the body independent of electric waves and electric current. Inventors of the present invention have been considering that the information can be communicated by electrostatic induction. Moreover, the inventors made a practical apparatus, and tested and confirmed that the communication mentioned above is possible.

Thus, in this embodiment, it is not necessary to install an antenna, a magnet, and a magnetic sensor etc. in the medical capsule device 100 and the apparatus outside the body 200. Therefore, it is possible to make the apparatus smaller, and to reduce strain on the patient. Moreover, there is no dead zone due to a direction of the electric field, and it is possible to detect accurately the position of the medical capsule device 100.

Furthermore, a transmission of the in vivo information and the signal for position detection from the medical capsule device 100 to the apparatus outside the body 200 has been described above. However, it is not restricted to this. It is also possible to make a structure in which an electric power signal and a driving signal for CCD are transmitted from the apparatus outside the body 200 to the medical capsule device 100.

Second embodiment

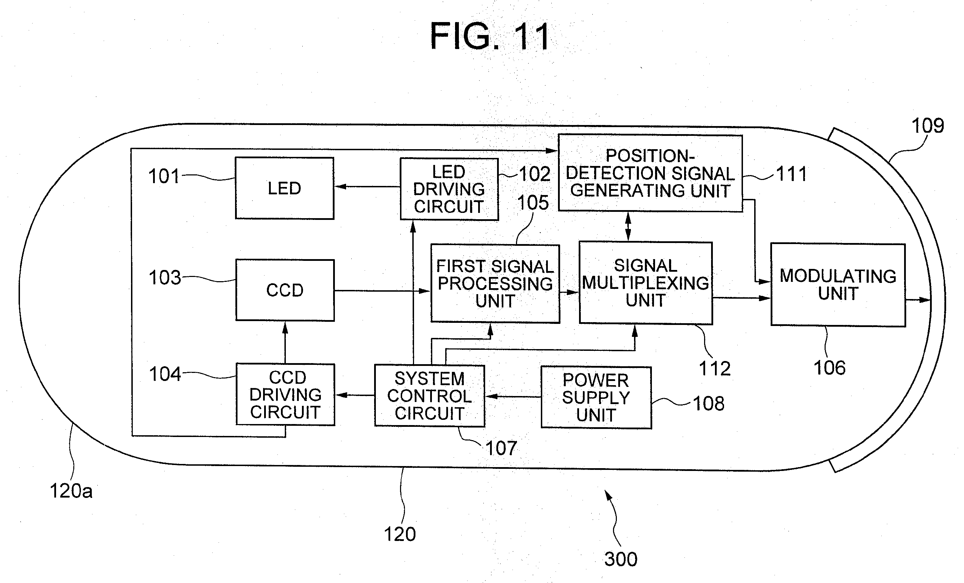

Next, an in-body position detecting system according to a second embodiment of the present invention will be described below. Same reference numerals are assigned to the components which are same as in the first embodiment, and the description to be repeated is omitted. Fig. 11 shows a schematic structure of a medical capsule device 300 according to the second embodiment.

In this embodiment, a point that instead of the signal synchronizing unit 110, a signal multiplexing unit 112 is provided differs from the first embodiment. The signal multiplexing unit 112 superimposes the signal for position detection on the in vivo information signal, and outputs to the modulating unit 106.

Further, at a side of the apparatus outside the body 200, the demodulating unit 203, based on the change in the electric potential of the second pads 201a, 201b, and 201c, outputs the in vivo information signal to the second signal processing unit 206. Moreover, the demodulating unit 203, based on the change in the electric potential of the second pads 201a, 201b, and 201c, outputs the signal for position detection which is demodulated, to the position calculating unit 204. Further, by a procedure similar to the first embodiment, it is possible to calculate the three-dimensional position information of the medical capsule device 100.

Here, the in vivo information signal is a video signal. Further, the signal multiplexing unit 112 superimposes the signal for position detection on the video signal. At this time, it is possible to modulate and superimpose the signal for position detection by a signal of a frequency higher than a frequency of the video signal.

Third embodiment

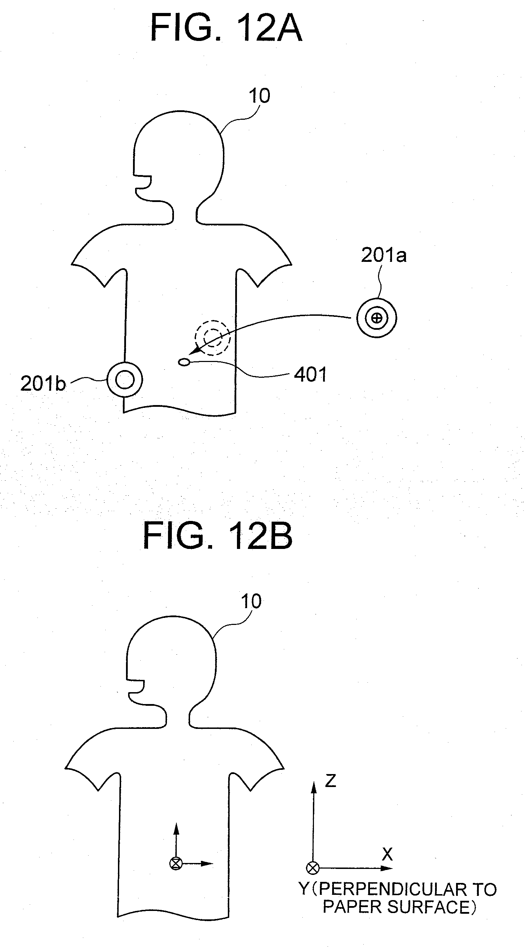

Next, an in-body position detecting system according to a third embodiment of the present invention will be described below. Same reference numerals are assigned to the components which are same as in the first embodiment, and the description to be repeated is omitted. Figs. 12A and 12B are diagrams showing the living body 10 and the second pad 201a which is a reference of the in-body position detecting system according to the third embodiment.

At least one of the second pads provided in the apparatus outside the body mentioned above is the pad 201a which becomes the reference for the position detection. The second pad which becomes the reference is attached to a predetermined position, such as a peculiar part of the living body. As a peculiar part of the living body, a navel, a nipple, and a backbone can be used. In this embodiment, as a peculiar part, a navel 401 is used.

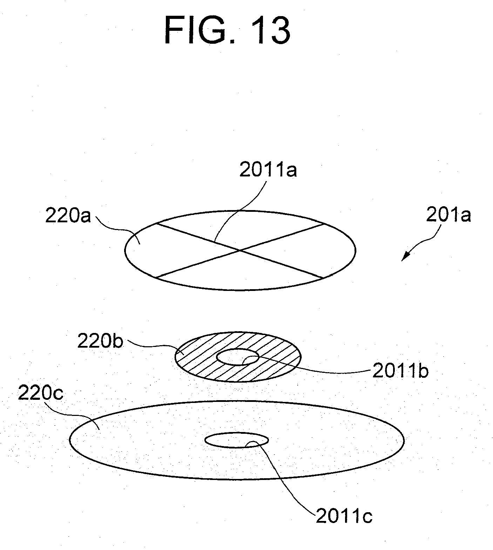

Fig. 13 is an exploded view showing a perspective structure of the second pad 201a which becomes the reference. The other second pads 201b and 201c also have a structure similar to the second pad 201a. Since the second pad 201a makes a close contact with the body surface, the second pad 201a has a structure in which the thin film 220b made of a material such as platinum (Pt) and gold (Au) is sandwiched by the substrate 220a such as a resin film and a ribbon.

For example, through holes 2011b and 2011c are formed near a center of the circular shaped thin film 220b and the insulating thin-film 220c. The substrate 220a is a substantially transparent material, and reference line 2011a which are substantially orthogonal to at least one surface, are drawn. The reference lines 2011a intersect near a center of the substrate 220a.

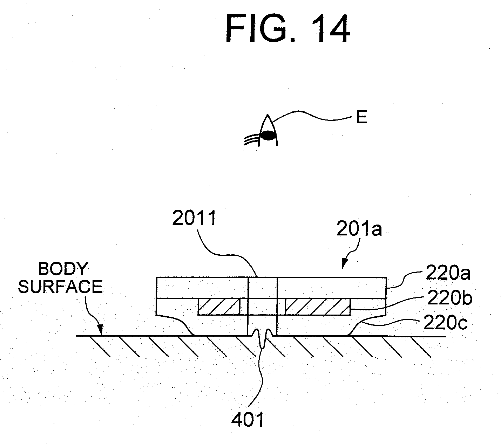

The substrate 220a, the thin film 220b, and the insulating thin-film 220c are stuck such that a central position of the through hole 2011b in the thin film 220b, a central position of the through hole 2011c in the insulating thin-film 220c, and a point of intersection of the reference line 2011a of the substrate 220a coincide. Accordingly, a position-adjustment mark 2011 (Fig. 14) is formed.

The position-adjustment mark 2011 corresponds to a position adjusting section. The position-adjustment mark 2011 is used for performing a relative position adjustment with respect to the navel 401 which is the predetermined position of the living body 10.

Fig. 14 shows a cross-sectional structure of the second pad 201a which becomes the reference. On a portion of the second pad 201a which makes a contact with the surface of the body, the insulating thin-film 220c of a silicon resin etc. is formed. It is desirable that the insulating thin-film 220c which makes a contact with the surface of the body has a thickness which enables to detect an electric potential of the surface of the body by the second signal processing unit 206. The thickness of the insulating thin-film 220c is not more than 1 mm for example.

Moreover, a gel or oil may be applied between the surface of the living body 10 and the second pad 201a. Accordingly, it is possible to improve further an adhesion between the second pad 201a and the surface of the body.

Accordingly, based on a three-dimensional relative position of each of the pads 201a, 201b, and 201c, and the medical capsule device 100, it is possible to calculate a three-dimensional absolute position of the medical capsule device 100 in terms of coordinates as shown in Fig 12B in which the navel 401 is let to be the reference.

Without sticking around a position change such as a change in a posture and an amount of movement of the medical capsule device 100, it is possible to associate the absolute position and a direction of observation of the medical capsule device 100 with general information and the in vivo information of the living body 10. Therefore, it is possible to know the position of the medical capsule device 100 in a manner which makes it easy to use.

Further, an observer E, while observing the navel 401 through the through hole 2011b and the through hole 2011c can attach the pad 201a for reference by adjusting with the point of intersection of the reference line 2011a and the substantial center of the navel 401. Accordingly, it is possible to attach the pad 201a for reference at the same position with a favorable repeatability. Therefore, it is possible to perform easily a comparison of the in vivo information at different times of examination.

The position adjusting section in this embodiment uses the through holes 2011b and 2011c, and the reference line 2011 which is a cross line. However, it is not restricted to this provided that it is a structure in which it is possible to perform the relative position adjustment of the pad which is a reference, with respect to the predetermined position of the living body 10. For example, a mark for position adjustment may be installed on a surface of the pad, or a notch or a projection may be provided on an outer side of the pad.

Moreover, in each embodiment described above, it is desirable to calculate the position information, upon taking into consideration an attenuation of a signal when propagated in the living body 10. Accordingly, it is possible to find even more accurate position of the medical capsule device 100.

Furthermore, as it has been mentioned above, when the demodulating unit 203 exclusively for each of the second pads 201a, 201b, and 201c is connected to the second pads 201a, 201b, and 201c respectively, the switching unit 202 may not be provided. At this time, for all the second pads 201a, 201b, and 201c, it is possible to find the distance from the medical capsule device 100. At this time, for example, it is possible to use a least-square method in the calculation for the position detection. Accordingly, there is an advantage that it is possible to reduce further an error in deriving the position of the medical capsule device 100.

Furthermore, in the calculation for the position detection, a structure in which the position detection is performed for a plurality of times, and an average of the positions acquired is taken, may be adopted.

Moreover, the medical capsule device in each of the embodiments mentioned above is structured to take images of inside of the living body by providing an LED and a CCD etc. However, an apparatus introduced inside the body which is introduced inside the body to be examined is not restricted to such structure, and may be let to be an apparatus which acquires other in vivo information such as information of temperature and pH of the body to be examined for example. Furthermore, the medical capsule device 100 may be structured to include an oscillator, and let to acquire an ultrasonic image of inside of the living body 10. In addition, a structure may be such that a plurality of information is acquired from the in vivo information.

Moreover, in each of the embodiments described above, as a body to be examined, an example of examining and observing a human body is shown. However, the present invention is not restricted to this, and an industrial product for example, may be used as a body to be examined.

According to the present invention it is possible to provide an in-body position detecting system which is capable of detecting accurately and without a dead zone due to a direction of the electric field, a position, without a need of disposing an antenna, a magnet, and a magnetic field detecting sensor in an apparatus outside the body to be examined, and which reduces the strain on the patient, as a size of the apparatus inside the body to be examined can be made smaller.

INDUSTRIAL APPLICABILITY

As it has been mentioned above, an in-body position detecting system of the present invention is small sized, and is useful in reducing a strain on a patient (living body), and in a case of acquiring position information thereof.

**Claims**

1. An in-body position detecting system, comprising:

   an apparatus introduced inside the body to be examined, which is introduced inside the body; and
   an apparatus outside the body which is disposed outside the body to be examined, and performs a communication with the apparatus introduced inside the body to be examined, wherein
   the apparatus introduced inside the body to be examined includes at least a first pad, and
   the apparatus outside the body includes a plurality of second pads, and
   for performing transceiving of a signal between the first pad and the second pad, at least one of the apparatus introduced inside the body to be examined and the apparatus outside the body includes a modulating unit which applies a voltage to the pad of one of the apparatuses, upon modulating the signal, and
   the other apparatus includes a demodulating unit which demodulates the signal based on a change in an electric potential of the pad of the other apparatus, and further comprising:

      a signal generating unit which transmits a signal for position detection; and
      a position calculating unit which calculates a position of the in-body position detecting system, based on a magnitude of signal strength of the signal for position detection which is demodulated based on the change in the electric potential at the plurality of second pads.

2. The in-body position detecting system according to claim 1 wherein
   the signal generating unit transmits the signal for position detection, by synchronizing with in vivo information signal.

3. The in-body position detecting system according to claim 2, wherein
   the signal for position detection is generated by using a clock signal for control of at least one of the apparatus introduced inside the body to be examined and the apparatus outside the body.

4. The in-body position detecting system according to claim 1, wherein
   the signal generating unit, when not transmitting the in vivo information signal, transmits the signal for position detection.

5. The in-body position detecting system according to claim 4, wherein
   the in vivo information signal is a video signal, and
   the signal generating unit transmits the signal for position detection in an interval of a blank signal for a vertical synchronization of the video signal.

6. The in-body position detecting system according to claim 1, wherein
   the signal generating unit transmits the signal for position detection upon multiplexing with the in vivo information signal.

**7.** The in-body position detecting system according to claim 6, wherein
the apparatus introduced inside the body to be examined includes an imaging section which takes images of a portion to be examined of the body to be examined, and outputs at least a video signal, and
the apparatus outside the body demodulates the video signal, and
the signal for position detection is superimposed on the video signal.

**8.** The in-body position detecting system according to claim 6, wherein
the signal for position detection is generated by using a clock signal for control of at least one of the apparatus introduced inside the body to be examined and the apparatus outside the body.

**9.** The in-body position detecting system according to claim 1, wherein
at least one of the second pads included in the apparatus outside the body is a pad which becomes a reference for the position detection.

**10.** An in-body position detecting system comprising:

an apparatus introduced inside the body to be examined which is introduced inside the body to be examined; and
an apparatus outside the body which is disposed outside the body, and performs a communication with the apparatus introduced inside the body to be examined, wherein
the apparatus introduced inside the body to be examined includes at least a first pad, and
the apparatus outside the body includes a plurality of second pads, and
for performing transceiving of a signal between the first pad and the second pad, at least one of the apparatus introduced inside the body to be examined and the apparatus outside the body includes a modulating unit which applies a voltage to the pad of one of the apparatuses upon modulating the signal, and
the other apparatus includes a demodulating unit which demodulates the signal based on a change in an electric potential of the pad of the other apparatus, and further comprising:

a signal generating unit which transmits a signal for position detection; and
a position calculating unit which calculates a position of the in-body position detecting system, based on a magnitude of a signal strength of the signal for position detection which is demodulated based on the change in the electric potential at the plurality of second pads, wherein
at least one of the second pads included in the apparatus outside the body is a pad which becomes a reference for position detection, and
the pad which becomes the reference has a position adjusting section for adjusting a relative position with respect to a predetermined position of the body to be examined.

**11.** The in-body position detecting system according to claim 10, wherein
the signal generating unit transmits the signal for position detection upon synchronizing with in vivo information signal.

**12.** The in-body position detecting system according to claim 11, wherein
the signal for position detection is generated by using a clock signal for control of at least one of the apparatus introduced inside the body to be examined and the apparatus outside the body.

**13.** The in-body position detecting system according to claim 10, wherein
the signal generating unit, when not transmitting the in vivo information signal, transmits the signal for position detection.

**14.** The in-body position detecting system according to claim 13, wherein
the in vivo information signal is a video signal, and
the signal generating unit transmits the signal for position detection in an interval of a blank signal for a vertical synchronization of the video signal.

**15.** The in-body position detecting system according to claim 10, wherein
the signal generating unit transmits the signal for position detection upon multiplexing with the in-vivo information signal.

**16.** The in-body position detecting system according to claim 15, wherein
the apparatus introduced inside the body to be examined includes an imaging section which takes images of a portion to be examined of the body to be examined, and outputs at least a video signal, and
the apparatus outside the body demodulates the video signal, and
the signal for position detection is superimposed on the video signal.

**17.** The in-body position detecting system according to claim 15, wherein
the signal for position detection is generated by using a clock signal for control of at least one of the apparatus
introduced inside the body to be examined and the apparatus outside the body.

# FIG. 1

# FIG. 2

# FIG. 3

FIG. 4

200

210

201a

201b

201c

202 — SWITCHING UNIT

203 — DEMODULATING UNIT

204 — POSITION CALCULATING UNIT

205 — POSITION OUTPUTTING UNIT

206 — SECOND SIGNAL PROCESSING UNIT

207 — RECORDING UNIT

208 — POWER SUPPLY UNIT

209 — DISPLAY UNIT

# FIG. 5

# FIG. 6

# FIG. 7A

```
                    ┌──────────┐
                    │  START   │
                    └──────────┘
                         │
                         ▼
        S701 ┌──────────────────────┐
             │   OUTPUT CCD          │
             │   DRIVING SIGNAL      │
             └──────────────────────┘
                         │
                         ▼
        S702 ┌──────────────────────┐
             │ CCD ACQUIRES INFORMATION │
             │ AND OUTPUTS TO SIGNAL    │
             │   PROCESSING UNIT        │
             └──────────────────────┘
                         │
                         ▼
   S703 ┌────────────────────────────────┐
        │ GENERATE IN VIVO INFORMATION SIGNAL AND │
        │ OUTPUT TO SIGNAL SYNCHRONIZING UNIT     │
        └────────────────────────────────┘
                         │
                         ▼
   S704 ┌────────────────────────────┐
        │ GENERATE SIGNAL FOR POSITION │
        │ DETECTION AND OUTPUT TO SIGNAL │
        │   SYNCHRONIZING UNIT          │
        └────────────────────────────┘
                         │
                         ▼
   S705 ┌────────────────────────────┐
        │   SYNCHRONIZE IN VIVO        │
        │ INFORMATION SIGNAL AND       │
        │ SIGNAL FOR POSITION          │
        │ DETECTION, AND OUTPUT        │
        └────────────────────────────┘
                         │
                         ▼
                       ( A )
```

# FIG. 7B

(A)

S707

S706

**S707**
MODULATE IN VIVO
INFORMATION SIGNAL,
AND APPLY VOLTAGE TO
FIRST PAD

**S706**
MODULATE SIGNAL FOR
POSITION DETECTION,
AND APPLY VOLTAGE
TO FIRST PAD

S708
CHANGE IN ELECTRIC
POTENTIAL OF SIGNAL FOR
POSITION DETECTION
IN SECOND PAD

(B)

S709
DEMODULATE SIGNAL FOR
POSITION DETECTION

S710
CALCULATE POSITION

DISPLAY POSITION

S711

# FIG. 7C

Ⓐ

S712

Ⓑ

DEMODULATE IN VIVO INFORMATION SIGNAL

PERFORM SIGNAL PROCESSING — S713

S714

OUTPUT IN VIVO INFORMATION TO DISPLAY UNIT

S716

OUTPUT IN VIVO INFORMATION TO RECORDING UNIT

DISPLAY IN VIVO INFORMATION

RECORD IN VIVO INFORMATION

S715

S717

END

S718

STATE AT PREVIOUS TIME WAS BETTER

COMPARE STATE OF DEMODULATED OUTPUT SIGNAL WITH PREVIOUS ONE

STATE AT THIS TIME IS BETTER

S719 — CHANGE MODULATION FREQUENCY

# FIG. 8A

```
                    ┌─────────────┐
                    │    START    │
                    └─────────────┘
                           │
                           ▼
        ┌──────────────────────────────────────┐
        │         LET DISTANCE BETWEEN          │
        │     MEDICAL CAPSULE DEVICE 100        │──── S801
        │      AND PADS 201a, 201b, AND         │
        │         201c BE La, Lb, AND Lc        │
        │             RESPECTIVELY              │
        └──────────────────────────────────────┘
                           │
                           ▼
        ┌──────────────────────────────────────┐
        │         LET ELECTRIC POTENTIAL        │──── S802
        │    GENERATED IN PADS 201a, 201b, AND  │
        │      201c BY SIGNAL FOR POSITION      │
        │       DETECTION BE Va, Vb, AND Vc     │
        │             RESPECTIVELY              │
        └──────────────────────────────────────┘
                           │
                           ▼
    ┌──────────────────────────────────────────────┐
    │        LET POSITION COORDINATES OF PADS       │──── S803
    │   201a, 201b, AND 201c BE (ax, ay, az), (bx,  │
    │   by, bz), AND (cx, cy, cz) RESPECTIVELY, AND │
    │    POSITION COORDINATES OF PAD 109 OF MEDICAL │
    │         CAPSULE DEVICE 100 BE (Px, Py, Pz)    │
    └──────────────────────────────────────────────┘
                           │
                           ▼
                          (A)
```

# FIG. 8B

$(A)$

$$(Px-ax)^2 + (Py-ay)^2 + (Pz-az)^2 = La^2 \text{ (EXPRESSION 1)}$$
$$(Px-bx)^2 + (Py-by)^2 + (Pz-bz)^2 = Lb^2 \text{ (EXPRESSION 2)}$$
$$(Px-cx)^2 + (Py-cy)^2 + (Pz-cz)^2 = Lc^2 \text{ (EXPRESSION 3)}$$
ARE ESTABLISHED

— S804

$$Va \propto 1/La^n$$
$$Vb \propto 1/Lb^n$$
$$Vc \propto 1/Lc^n$$

— S805

(ax, ay, az), (bx, by, bz), AND (cx, cy, cz) ARE POSITIONS AT WHICH PADS 201a, 201b, AND 201c ARE STUCK, AND THESE POSITIONS ARE ALREADY KNOWN

— S806

$(B)$

# FIG. 8C

```
                    ( B )
                      │
                      ▼
```

CALCULATE POSITION COORDINATES (Px, Py, Pz) OF MEDICAL CAPSULE DEVICE 100 BY ACQUIRING ELECTRIC POTENTIALS Va, Vb, AND Vc GENERATED BY SIGNAL FOR POSITION DETECTION, AND SOLVING EQUATION BY SUBSTITUTING FROM EXPRESSION 1 TO EXPRESSION 3 — S807

FURTHER, LET POSITION COORDINATES OF ONE OF PADS BE ORIGIN (0, 0, 0), AND DEFINE COORDINATE SYSTEM SUCH THAT LEFT HAND SIDE OF BODY IS POSITIVE DIRECTION OF X, AND CALCULATE POSITION COORDINATES (Px, Py, Pz) OF MEDICAL CAPSULE DEVICE 100 WITH ONE OF PADS LET TO BE REFERENCE — S808

STICK ONE OF PADS TO PECULIAR PART OF BODY SUCH AS NAVEL, AND WITH THIS PECULIAR PART OF BODY AS REFERENCE, CALCULATE THREE DIMENSIONALLY, POSITION OF MEDICAL CAPSULE DEVICE 100 — S809

```
                    ( END )
```

# FIG. 9A

START

LET DISTANCE BETWEEN MEDICAL CAPSULE DEVICE 100 AND PADS 201a, 201b, AND 201c BE La, Lb, AND Lc RESPECTIVELY —— S901

MODULATE SIGNALS FOR POSITION DETECTION HAVING DIFFERENT TRANSMISSION TIMING AND FREQUENCY AND APPLY THESE MODULATED SIGNALS TO PADS 109 AND 130 —— S902

LET ELECTRIC POTENTIAL GENERATED IN PADS 201a, 201b, AND 201c BY SIGNAL FOR POSITION DETECTION BE (Va, Vb, Vc) AND (Va2, Vb2, Vc2) RESPECTIVELY —— S903

LET POSITION COORDINATES OF PADS 201a, 201b, AND 201c BE (ax, ay, az), (bx, by, bz), AND (cx, cy, cz) RESPECTIVELY, POSITION COORDINATES OF PAD 109 OF MEDICAL CAPSULE DEVICE 100 BE (Px, Py, Pz), AND POSITION COORDINATES OF PAD 130 BE (Px2, Py2, Pz2) —— S904

A

# FIG. 9B

(A)

S905

$(Px-ax)^2 + (Py-ay)^2 + (Pz-az)^2 = La^2$ (EXPRESSION 1)
$(Px-bx)^2 + (Py-by)^2 + (Pz-bz)^2 = Lb^2$ (EXPRESSION 2)
$(Px-cx)^2 + (Py-cy)^2 + (Pz-cz)^2 = Lc^2$ (EXPRESSION 3)
ARE ESTABLISHED

$Va \propto 1/La^n$
$Vb \propto 1/Lb^n$
$Vc \propto 1/Lc^n$

S906

(ax, ay, az), (bx, by, bz), AND (cx, cy, cz) ARE POSITIONS AT WHICH PADS 201a, 201b, AND 201c ARE STUCK, AND THESE POSITIONS ARE ALREADY KNOWN

S907

BY SIMILAR CALCULATION, IT IS POSSIBLE TO CALCULATE POSITION COORDINATES (Px2, Py2, Pz2) OF PAD 130 AND POSITION COORDINATES (Px, Py, Pz) OF PAD 109 OF MEDICAL CAPSULE DEVICE 100

IT IS POSSIBLE TO CALCULATE POSITION COORDINATES (Px, Py, Pz) OF PAD 109 OF MEDICAL CAPSULE DEVICE 100 BY ACQUIRING ELECTRIC POTENTIALS Va, Vb, AND Vc GENERATED BY SIGNAL FOR POSITION DETECTION, AND SOLVING EQUATION BY SUBSTITUTING FROM EXPRESSION 1 TO EXPRESSION 3

S908

(B)

# FIG. 9C

(B)

FURTHER, LET POSITION COORDINATES OF ONE OF
PADS BE ORIGIN (0, 0, 0), AND DEFINE COORDINATE
SYSTEM SUCH THAT LEFT HAND SIDE OF BODY IS
POSITIVE DIRECTION OF X, AND CALCULATE POSITION
COORDINATES (Px, Py, Pz) OF MEDICAL CAPSULE
DEVICE 100 WITH ONE OF PADS LET TO BE REFERENCE — S909

WHEN ONE OF PADS IS STUCK TO PECULIAR PART OF BODY
SUCH AS NAVEL, IT IS POSSIBLE TO CALCULATE THREE
DIMENSIONALLY POSITION OF MEDICAL CAPSULE
DEVICE 100 WITH PECULIAR PART OF BODY AS REFERENCE — S910

WHEN VECTOR OPERATION IS PERFORMED ON
POSITION COORDINATES (Px, Py, Pz) OF
PAD 109 OF MEDICAL CAPSULE DEVICE 100,
POSITION COORDINATES (Px2, Py2, Pz2) OF
PAD 130, IT IS POSSIBLE TO CALCULATE IN VIVO
POSITION OF MEDICAL CAPSULE DEVICE 100 — S911

END

# FIG. 10

# FIG. 11

EP 1 961 365 A1

## FIG. 12A

## FIG. 12B

Z

X

Y (PERPENDICULAR TO PAPER SURFACE)

# FIG. 13

2011a

220a

201a

220b

2011b

220c

2011c

# FIG. 14

<table>
<tr><td colspan="2"><strong>INTERNATIONAL SEARCH REPORT</strong></td><td>International application No.<br><br>PCT/JP2006/324134</td></tr>
</table>

| A. CLASSIFICATION OF SUBJECT MATTER |
|---|
| *A61B1/00(2006.01)i, A61B5/06(2006.01)i, A61B5/07(2006.01)i* |

According to International Patent Classification (IPC) or to both national classification and IPC

| B. FIELDS SEARCHED |
|---|

Minimum documentation searched (classification system followed by classification symbols)
A61B1/00, A61B5/06, A61B5/07

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| Y | JP 2005-260751 A (Olympus Corp.),<br>22 September, 2005 (22.09.05),<br>Par. Nos. [0009] to [0012], [0019] to [0027];<br>Figs. 2, 3<br>& WO 2005/065525 A | 1-17 |
| Y | JP 2005-87726 A (Biosense Webster, Inc.),<br>07 April, 2005 (07.04.05),<br>Par. Nos. [0019], [0020]; Fig. 1A<br>& US 2005-27330 A1 & EP 1502540 A1<br>& CA 2475917 A | 1-17 |
| A | JP 2003-19111 A (Given Imaging Ltd.),<br>21 January, 2003 (21.01.03),<br>Full text; all drawings<br>& US 2002-173718 A1 & EP 1260176 A2 | 1-17 |

| ☐ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>22 December, 2006 (22.12.06) | Date of mailing of the international search report<br>09 January, 2007 (09.01.07) |
|---|---|
| Name and mailing address of the ISA/<br>Japanese Patent Office | Authorized officer |
| Facsimile No. | Telephone No. |

Form PCT/ISA/210 (second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003019111 A
- JP 2005192631 A